# EUROPÄISCHE PATENTANMELDUNG

(11) **EP 1 046 707 A1**
(43) Veröffentlichungstag der Anmeldung: **25.10.2000**
(21) Anmeldenummer: 00108707.1
(22) Anmeldetag: 22.04.2000
(51) Int. Cl.: C12M 3/02, C12M 1/26

(54) **Bioreaktor umfassend einen Medienablauf und einen Rührer sowie Rührer und Medienablauf**

(30) Priorität: 22.04.1999 DE 19918409
(71) Anmelder: FORSCHUNGSZENTRUM JÜLICH GMBH, 52425 Jülich (DE)
(72) Erfinder: Schmidt, Sebastian, 52428 Jülich (DE); Eisenkrätzer, Detlef, 79540 Lörrach (DE); Biselli, Manfred, Dr., 52428 Jülich (DE); Wandrey, Christian, Prof., 52428 Jülich (DE)

(57) **Zusammenfassung**

Die Erfindung betrifft einen Bioreaktor (1) umfassend einen Medienablauf (10) und einen Rührer sowie einen Rührer und einen Medienablauf und einen Filter zum Zurückhalten von sich in der Flüssigkeit befindenden Zellen der erfindungsgemäß dadurch gekennzeichnet ist, daß sein Rührer zum Einsatz kommt, der dicht über dem Boden des Bioreaktors (1) liegt und der Bioreaktor (1) einen Medienablauf (10) besitzt, der dicht über dem Rührer (13) endet und vorzugsweise als Konus (11) ausgebildet ist und mit einem Filter (12) ausgestattet ist, welches in wesentlichen parallel zum Boden des Bioreaktors (1) verläuft und einen geringen Abstand zum Rüher (1) hat. Vorteilhafterweise ist der Rührer (13) in Rotationsrichtung in einem Winkel von 45° abgewinkelt, so daß er bei der Rotation eine Aufwärtsströmung erzeugt.

Der Bioreaktor ist insbesondere für die Züchtung von wertvollen Zellen geeignet, die ein kleines Reaktorvolumen erfordern. Weiterhin ist der Rührer (13) besonders zellschonend.

## Beschreibung

Die Erfindung betrifft einen Bioreaktor umfassend einen Medienablauf und einen Rührer nach dem Oberbegriff des Anspruchs 1 sowie einen Rührer und einen Medienablauf.

In der Biotechnologie werden Bioreaktoren eingesetzt, in denen Zellen vermehrt werden können. Diese Bioreaktoren bestehen regelmäßig aus Rührkesseln, die mit Medienzulauf und Medienablauf sowie Probenahmestutzen ausgestattet sind. Weiterhin sind diese Bioreaktoren in der Regel mit Meßgeräten wie pH-Meßgeräten, Sauerstoffkonzentrationsmeßgeräten und Öffnungen für Luft- oder Sauerstoffzufuhr ausgestattet. In diesen Bioreaktoren werden die zu vermehrenden Zellen in einem Medium gehalten, welches Substrat enthält, damit sich die Zellen vermehren können. Die Substratkonzentration wird dabei in der Regel durch einen Medienzulauf und eine Medienabführung in einem stationären Konzentrationsverhältnis gehalten. Nach einer bekannten Methode geschieht dies so, daß durch ein Medienzulauf Substrat in einer Flüssigkeit eingespeist wird und die gleiche Volumenmenge über einen Medienablauf abgeführt wird. Der Medienablauf erfolgt dabei über einen Spinfilter, welcher an einen Rührer gekoppelt ist, der gleichzeitig eine intensive Verrührung der Zellen mit dem Medium bewirkt. Die Porengröße des Spinfilters ist meist größer als der Durchmesser einer Zelle, jedoch wird durch die schnelle Rotation des Spinfilters ein Eindringen der Zellen in den Medienablauf weitgehend verhindert. Hat eine entsprechende Vermehrung der Zellen stattgefunden, so daß die Zellpopulationsdichte einen bestimmten Grenzwert erreicht hat, so ist es bei diesen Reaktoren in der Regel möglich, das Volumen des Mediums zu erhöhen, so daß eine Verdünnung der Zellen stattfindet. Dies kann durch zusätzlichen Medienzulauf erfolgen. Zwar werden diese Vorrichtungen vielen Erfordernissen des Gebrauchs gerecht, jedoch finden ihre Anwendungen Grenzen. Manche zu vermehrenden Zellen stehen nur in geringen Mengen zur Verfügung. Dies gilt beispielsweise für Spenderzellen, die in kleinen Zellkonzentrationen auftreten, da sie nicht in beliebigen Mengen einem lebenden Organismus entnommen werden können. Beispiele für derartige Primärzellen sind hämatopoetische Zellen. Da derart wertvolles Zellmaterial nur in geringen Mengen zur Verfügung steht, ist es erforderlich, diese Zellen in einem verhältnismäßig kleinen Medienvolumen von ca. 40 ml heranzuzüchten. Erst bei entsprechender Zunahme der Zellpopulation durch Zellteilung ist es möglich, die Population in einem größeren Medienvolumen zu halten. Die nach dem Stand der Technik verfügbaren Bioreaktoren sind jedoch für die Heranzüchtung derart kleiner Zellzahlen ungeeignet.

Es ist daher die Aufgabe der Erfindung einen Bioreaktor zu schaffen, der es ermöglicht, kleinste Zellmengen und damit verbunden kleine Medienvolumina zu kultivieren. Weiterhin soll der Reaktor derart ausgestaltet sein, daß keine wesentliche Beschädigung der wertvollen Zellen während der Züchtung stattfindet.

Ausgehend vom Oberbegriff des Anspruchs 1 wird die Aufgabe erfindungsgemäß gelöst mit den im kennzeichnenden Teil des Anspruchs 1 angegebenen Merkmalen.

Mit dem erfindungsgemäßen Bioreaktor ist es nunmehr möglich, geringe Zellmengen wertvollen Zellmaterials auf schonende Art und Weise zu vermehren.

Vorteilhafte Weiterbildungen der Erfindung sind in den Unteransprüchen angegeben.

Die Zeichnungen zeigen einen erfindungsgemäßen Bioreaktor sowie Einzelteile davon in schematischer Form.

Es zeigt
- Figur 1:: einen erfindungsgemäßen Bioreaktor,
- Figur 2:: einen erfindungsgemäßen Medienablauf mit Filter,
- Figur 3, 3a:: einen erfindungsgemäßen Rührer.

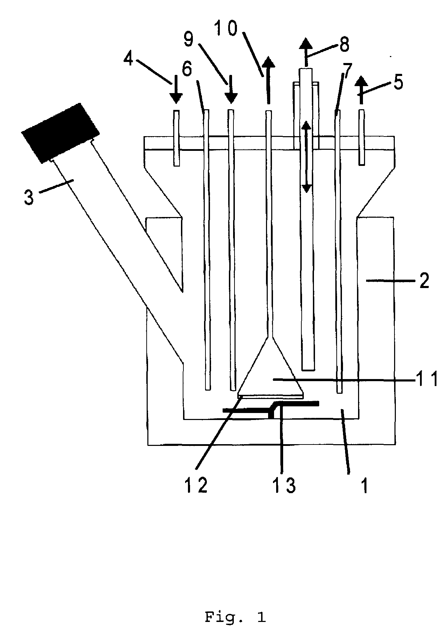

Figur 1 zeigt einen Bioreaktor 1, der sich in einem Wärmemantel 2 befindet. Der Bioreaktor 1 besitzt einen Probenahmestutzen 3, einen Gaseinlaß 4, einen Gasauslaß 5, einen pH-Meßfühler 6, einen pO₂-Meßfühler 7, eine Füllstandsregelung 8, einen Medienzulauf 9 und einen Medienablauf 10. Der Medienablauf 10 weitet sich an seinem unteren Ende zu einem Konus 11 auf, welcher einen Filter 12 aufnimmt. Unterhalb des Konus 11 mit dem Filter 12 befindet sich ein Magnetrührkern 13.

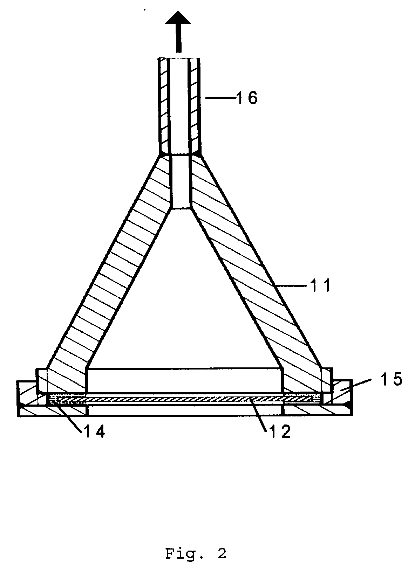

Figur 2 zeigt eine Detaildarstellung des unteren Bereiches des Medienablaufes 10. In ihr ist der Konus 11 mit dem Filter 12 zu sehen, welches über eine Dichtung 14 mit einer Überwurfmutter 15 an den Konus 11 angebracht ist.

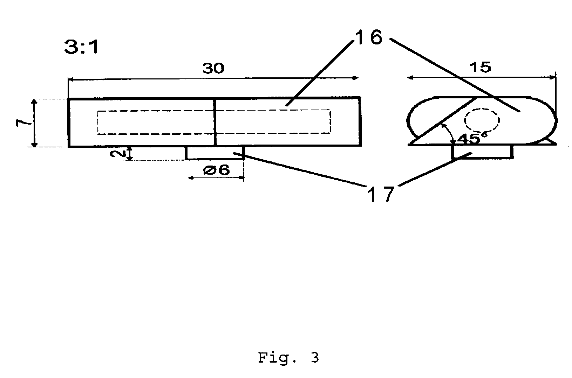

Die Figuren 3 und 3a zeigen Ansichten des erfindungsgemäßen Magnetrührkerns 13. In Figur 3 ist eine Projektion der Seitenansicht abgebildet. In ihr ist der rühraktive Korpus 16 zu sehen, welcher sich auf einer Auflagefläche 17 befindet, die mittig angeordnet ist.

Figur 3a zeigt eine Projektion der Schmalseite des Magnetrührkerns 13 auf die Rotationsachse, wobei die Rotationsachse den Korpus 16 des Magnetrührkerns 13 mittig teilt. Die Projektion der Figur 3a zeigt, daß die Hälften des Korpus 16 einen Querschnitt haben, welcher auf einer Seite abgerundet ist und auf der gegenüberliegenden Seite mit der Unterkante, welche an die Auflagefläche 17 grenzt, einen Winkel von 45° ausbildet, welcher eine Schräge ausbildet, die in der halben Breite des Korpus 16 endet. Die andere Hälfte des Korpus 16 ist entgegengesetzt so ausgebildet, daß beim Drehen des Magnetrührkerns 13 eine Drehsymmetrie auftritt.

Der in der Figurenbeschreibung vorgestellte Magnetrührkern 13 ist beispielhaft für den erfindungsgemäßen Rührer, der als Arbeitsbegriff alle Antriebsmittel umfassen soll, die propellerartig ausgebildet sind und eine aufwärtsgerichtete Strömung erzeugen. Diese können auch Propeller mit Rührblättern sein, die konservativ durch eine Welle angetrieben werden, welche beispielsweise durch den Boden des Bioreaktors 1 in den Reaktorraum tritt. Der Begriff Rührer umfaßt dabei keine Teile, wie Motor und Welle, sondern bezieht sich auf das Element, welches durch seine Gestalt eine Rotationsbewegung in der Flüssigkeit bewirkt, also einen Magnetrührkern 13 oder einen Propeller mit Rührblättern. Sowohl die Propeller als auch der Magnetrührkern 13 können mehrblättrig ausgebildet sein. In einem Grenzfall kann der Rührer scheibenförmig ausgebildet sein und eine Vielzahl von Faltungen aufweisen, die eine nach oben gerichtete Strömung erzeugen. Der Rührer kann beispielsweise 1,2,3,4,5,6 u.s.w Blätter aufweisen und befindet sich dicht über dem Boden des Bioreaktors 1 bzw. liegt im Falle des Magnetrührkerns 13 auf dem Boden auf.

Bei Betrieb wird der Bioreaktor 1 mit der bestehenden Zellpopulation im Nährmedium beschickt. Der Wärmemantel 2 wird auf die zur Kultivierung notwendige Temperatur temperiert. Der Gaseinlaß 4 sowie der Gasauslaß 5 gewährleisten eine Belüftung des Bioreaktors 1, die durch den pO₂-Meßfühler 7 reguliert werden kann. Zur Kontrolle wird der pH-Meßfühler 6 eingeschaltet. Ein Magnetrührer bringt den Magnetrührkern 13 in Bewegung, welcher für eine kontinuierliche Durchmischung der Zellen mit dem Nährmedium sorgt. Durch den Verbrauch des Nährmediums entsteht ein Bedarf an der Zufuhr von neuen Nährstoffen. Diese Zufuhr geschieht durch den Medienzulauf 9. In gleichem Ausmaß, in dem die Zugabe des Medienvolumens erfolgt, kann durch den Medienablauf 10 eine Entnahme von Medium durch Absaugen erfolgen. Die Regulierung kann dabei durch den Füllstandsregler 8 vorgenommen werden. Während dieses Austausches von Nährmedium besteht das Erfordernis, daß die wertvollen Stammzellen in der Nährflüssigkeit im Reaktor verbleiben sollen. Vorzugsweise ist der Medienablauf 10 mit dem Konus 11 ausgestattet, welcher an dem der Bodenseite zugerichteten Ende einen Filter aufweist, der die wertvollen Zellen zurückhält. Das Filtergewebe ist dabei derart ausgestaltet, daß die Maschenweite kleiner ist als der Durchmesser einer Zelle. Somit wird ein Zurückbleiben der Zellen im Nährmedium des Bioreaktors 1 gewährleistet. Durch diese Anordnung ist es möglich, das Medienvolumen in dem Reaktor so klein zu halten, daß auch geringe Zellmengen kultiviert werden können, da der Füllstand aufgrund der tiefen Lage des Filters 12 niedrig gehalten werden kann. Der Konus 11 mit dem Filter 12 ist dabei so dicht über dem Magnetrührkern 13 daß die durch den Magnetrührkern 13 hervorgerufene Radialströhmung eine Ablösung von eventuell an dem Filter 12 anhaftenden Zellen bewirkt und befindet sich möglichst direkt über dem Magnetrührkern 13. Die konusartige Ausbildung des Medienablaufes 10 ist nicht zwingend notwendig, jedoch ist sie vorteilhaft, da die durch das Filter 12 abgezogene Flüssigkeit bei einem Engen Medienablauf 10 wegen dem kleinen Querschnitt und damit verbunden kleinen Totvolumen, den Reaktor schnell verläßt. Als Filter 12 kann ein durch die Dichtung 14 abgedichtetes Metallfiltergewebe eingesetzt werden, welches von der Maschenweite so gewählt ist, daß die Zellen das Filter 12 gerade nicht passieren können. Alle Einheiten, wie pH-Meßfühler 6, pO₂-Meßfühler 7, Gaseinlaß 4, Füllstandsregelung 8, können so dicht gepackt werden und dabei in das Medienvolumen eindringen, daß ein Ausgangsvolumen von ca. 40 ml für die Zellproben ermöglicht wird. Durch die schnelle Rotation des Magnetrührkerns 13 wird ein Anhaften der wertvollen Zellen am Filter 12 verhindert. In einer bevorzugten Ausführungsform hat daher der Magnetrührkern 13 in etwa die gleiche Länge wie der Durchmesser des Filters 12. In einer weiteren vorteilhaften Ausgestaltung der Erfindung hat der Korpus 16 des Magnetrührkerns 13 eine derartige Geometrie, daß er eine Aufwärtsströmung des Nährmediums zum Filter hin bewirkt. Diese Aufwärtsströmung wird durch eine Abwinkelung im Winkel von ca. 45° des Korpus 16 bewirkt. Jedoch muß der Winkel nicht zwingend 45° betragen, sondern kann auch nach oberen und unteren Werten davon abweichen. Entscheidend ist jedenfalls, daß durch eine Abwinkelung eine aufwärts gerichtete Strömung erzeugt wird. Abweichend von dem konkret dargestellten Beipsiel muß die Schräge, die durch den Winkel gebildet wird nicht zwingend in den halben Breite des Korpus 16 enden, sondern kann auch bei z.B. 2/3 der Breite enden. Wie in Figur 3a zu erkennen ist, besitzt der beispielhaft dargestellte Rührer 13 folgende Merkmale:
a) er ist vertikal zur Längsachse mittig in zwei Hälften geteilt, wobei die Teilung die Rotationsachse darstellt,
b) jede Hälfte ist auf der der Rotationsrichtung zugewandten Seite von unten nach oben verlaufend mindestens in einem Teilbereich entlang des Radius des Rührers in einem Winkel geneigt.
c) jede Hälfte ist auf der der Rotationsrichtung abgewandten Seite mindestens einem Teilbereich entlang des Radius des Rührers abgerundet.

Auf der dem Winkel gegenüberliegenden Seite des Rührkerns 13 ist der Korpus 16 vorzugsweise abgerundet, wodurch die wertvollen Zellen geschont werden, da die Turbulenzen an der der Rotationsrichtung abgewandten Seite vermindert werden, was für die Zellen streßfreier ist. Die Anschrägung von ca. 45° des Magnetrührkerns 13 verhindert eine Sogwirkung über dem Rührer 13 und bewirkt statt dessen eine aufwärts gerichtete Strömung des Medienvolumens. Diese Strömung trägt zusätzlich dazu bei, daß sich möglicherweise an den Filter 12 anhaftende Zellen leicht ablösen können, wodurch wiederum ein Anhaften bzw. eine Verstopfungsgefahr oder ggf. ein Durchtritt von Zellen in den Medienablauf 10 verhindert wird. Ausgehend von der Rotationsachse des Rührers 13 muß die Anschrägung nicht über den gesamten Radius verlaufen, so kann beispielsweise um die Rotationsachse ein Teil von der Schrägung ausgespart sein, so daß nur die Randbereiche angeschrägt sind. Gleiches gilt für die Abrundungen. In einer weiteren vorteilhaften Ausgestaltungsform der Erfindung besitzt der Magnetrührkern 13 eine Auflagefläche 17, welche gegenüber der Projektionsfläche des Korpus 16 wesentlich verringert ist. Die Auflagefläche 17 befindet sich im Zentrum des Magnetrührkerns 13 und liegt auf der Rotationsachse. Somit ist die Kontaktfläche zwischen dem Reaktorboden und dem Magnetrührkern 13 gegenüber üblichen Magnetrührkernen deutlich verringert. Dies führt wiederum dazu, daß eine sehr geringe Reibungsfläche vorhanden ist, die zu eventuellen Beschädigungen von Zellen führen kann. Dieses ist insbesondere bei der Vermehrung von sehr wertvollen und seltenen Zellproben vorteilhaft.

Mit dem erfindungsgemäßen Bioreaktor 1 können nun wertvolle Zellen in kleinsten Mengen in kleinsten Volumina kontrolliert vermehrt werden. Der Reaktor kann mit kleinsten Volumina ohne Totzone betrieben werden. Die Verhinderung des Verblockens des Filters 12 durch vorhandene Querströmungen ermöglicht darüber hinaus einen sicheren Langzeitbetrieb des Bioreaktors 1. Haben sich die Zellen vermehrt, so daß eine höhere Populationsdichte vorliegt, so kann der Reaktor über den Medienzulauf 9 mit mehr Flüssigkeitsvolumen beschickt werden, so daß auch ein Betrieb mit größeren Füllvolumina möglich ist.

## Patentansprüche

1. Bioreaktor (1) umfassend einen Rührer, einen Medienablauf (10) und einen Filter (12) zum Zurückhalten von sich in einer Flüssigkeit befindenden Zellen,
dadurch gekennzeichnet,
daß ein Rührer zum Einsatz kommt, der dicht über dem Boden des Bioreaktors (1) liegt und der Bioreaktor einen Medienablauf (10) besitzt der dicht über dem Rührer (13) endet und mit einem Filter (12) ausgestattet ist, welches im wesentlichen parallel zum Boden des Bioreaktors (1) verläuft und einen geringen Abstand zum Rührer(13) hat.

2. Bioreaktor nach Anspruch 1,
dadurch gekennzeichnet,
daß sich der Medienablauf (10) an seinem unteren Ende zu einem Konus (11) aufweitet.

3. Bioreaktor nach Anspruch 1 oder 2,
dadurch gekennzeichnet,
daß der Filter (12) eine Maschenweite besitzt, die kleiner ist als die Größe der Zellen, welche sich in der Flüssigkeit befinden.

4. Bioreaktor nach einem der Ansprüche 1 bis 3,
dadurch gekennzeichnet,
daß der Filter (12) im wesentlichen den gleichen Durchmesser hat, wie der Rührer (13).

5. Bioreaktor nach einem der Ansprüche 1 bis 4,
dadurch gekennzeichnet,
daß er mit einem Rührer (13) ausgestattet ist, der folgende Merkmale besitzt:
a) er ist vertikal zur Längsachse mittig in zwei Hälften geteilt, wobei die Teilung die Rotationsachse darstellt
b) jede Hälfte ist auf der der Rotationsrichtung zugewandten Seite von unten nach oben verlaufend mindestens in einem Teilbereich entlang des Radius des Rührers (13) in einem Winkel geneigt.

6. Bioreaktor nach Anspruch 5,
gekennzeichnet durch folgendes Merkmal:
c) jede Hälfte ist auf der der Rotationsrichtung abgewandten Seite mindestens in einem Teilbereich entlang des Radius des Rührers (13) abgerundet.

7. Bioreaktor nach einem der Ansprüche 1 bis 6,
dadurch gekennzeichnet,
daß der Rührer (13) eine kleine Auflagefläche (17) besitzt, die sich unter der Rotationsachse auf der Seite befindet, die der Schräge, die durch den Winkel gebildet wird abgeneigt ist.

8. Rührer,
dadurch gekennzeichnet,
daß er folgende Merkmale besitzt:
a) er ist vertikal zur Längsachse mittig in zwei Hälften geteilt, wobei die Teilung die Rotationsachse darstellt
b) jede Hälfte ist auf der der Rotationsrichtung zugewandten Seite von unten nach oben verlaufend in mindestens in einem Teilbereich entlang des Radius des Rührers (13) in einem Winkel geneigt.

9. Rührer nach Anspruch 8,
dadurch gekennzeichnet,
daß jede Hälfte ist auf der der Rotationsrichtung abgewandten Seite mindestens in einem Teilbereich entlang des Radius des Rührers (13) abgerundet ist.

10. Rührer nach Anspruch 8 oder 9,
dadurch gekennzeichnet,
daß er eine kleine Auflagefläche (17) besitzt, die sich unter der Rotationsachse auf der Seite befindet, die der Schräge, die durch den Winkel gebildet wird, abgeneigt ist.

11. Medienablauf,
dadurch gekennzeichnet,
daß er aus einer Leitung besteht, die sich an ihrem unteren Ende zu einem Konus (11) aufweitet, der ein Filter (12) aufnimmt.
